# EUROPEAN PATENT APPLICATION

(11) **EP 2 644 705 A1**
(43) Date of publication of application: **02.10.2013**
(21) Application number: 12162564.4
(22) Date of filing: 30.03.2012
(51) Int. Cl.: C12Q 1/68

(54) **Biomarker for bladder cancer**

(71) Applicant: RWTH Aachen, 52062 Aachen (DE)
(72) Inventor: Dahl, Prof. Dr. rer. nat. Edgar, 4721 Kelmis (BE); Rose, Michael, 52070 Aachen (DE)
(74) Representative: Kröncke, Rolf

(57) **Abstract**

The present invention relates to new methods for diagnosing or identifying bladder cancer in a subject for predicting the clinical outcome or determining the treatment course in a subject afflicted with bladder cancer as well as for the stratification of the therapeutic regimen of a subject with bladder cancer and for monitoring the progress of bladder cancer in a subject. The methods are based on the determination of the level or amount of methylation of the promoter of the ECRG4- and/or the promoter of the ITIH5-gene in a sample of said subject. In addition, the present invention relates to the use of a kit in a method according to the present invention. Finally, the present invention relates to new biomarkers, namely, the promoter of the ECRG4-gene or the promoter of the ITIH5-gene; in particular, the level or amount of methylation of said promoters as biomarkers for bladder cancer.

## Description

The present invention relates to new methods for diagnosing or identifying bladder cancer in a subject for predicting the clinical outcome or determining the treatment course in a subject afflicted with bladder cancer as well as for the stratification of the therapeutic regimen of a subject with bladder cancer and for monitoring the progress of bladder cancer in a subject. The methods are based on the determination of the level or amount of methylation of the promoter of the ECRG4- and/or the promoter of the ITIH5-gene in a sample of said subject. In addition, the present invention relates to the use of a kit in a method according to the present invention. Finally, the present invention relates to new biomarkers, namely, the promoter of the ECRG4-gene or the promoter of the ITIH5-gene, in particular, the level or amount of methylation of said promoter as a biomarker for bladder cancer.

### Background of the invention

Screening and monitoring assays are essential for the diagnosis and management of diseases or disorders. In particular, samples based on body fluids of the subject under investigation for such applications have the advantage that it is convenient for said subject to provide a sample and the risk of side effects is extremely low. Therefore, compliance is improved compared to diagnosis based on clinical examinations of the human or animal body by e.g. surgery and methods practiced on the human or animal body.

Bladder cancer is one of the most common cancers in man. In bladder cancer malignant or cancer cells are formed in the tissues of the bladder. The bladder stores urine until it is passed out of the body. When the bladder is emptied during urination, the urine goes from the bladder to the outside of the body through the urethra. Basically, three types of bladder cancer are known. All of them begin in the cells in the lining of the bladder. The different types of bladder cancer are named to the type of cells that become malignant:
I. Transitional cell carcinoma is a type of cancer that begins in the cells in the innermost tissue layer of the bladder. This is the most common type of bladder cancer.
II. Squamous cell carcinoma is a type of cancer that begins in the squamous cells. Squamous cells are thin flat cells that may form in the bladder after long term infection or irritation.
III. Adenocarcinoma: This type of cancer begins in glandular cells that may form in the bladder after long term irritation and inflammation.

It is known that smoking, gender and diet can affect the risk of developing bladder cancer. Typical signs for bladder cancer include blood in the urine or pain during urination.

Today, various tests and procedures are used for diagnosing bladder cancer including CT scan, urinalysis, internal exam of the vagina and/or rectum, intravenous pyelogram and cystoscopy. Typically, cystoscopy is used for diagnosing and therapy control of bladder cancer. The cystoscopy has several advantages including high specificity and high sensitivity. However, cystoscopy requires invasive treatment of the subject and is quite cost expensive. That is, in cystoscopy, the physician looks inside the bladder and urethra to check for abnormal areas. A cystoscope is inserted through the urethra into the bladder. A cystoscope is a thin, tube-like instrument with a light and lens for viewing. It also includes tools to remove tissue samples for later diagnosis. Typically a biopsy is taken to check for signs of cancer. Another possibility is to base diagnosis on urine cytology by examining the urine under a microscope to check for abnormal cells. However, although cytology is very specific, i.e. a positive result is likely to be indicative for bladder cancer, cytology suffers from low sensitivity since the conclusion that a negative result exclude bladder cancer is not possible.

Today, some diagnostic marker molecules have been described including human complement factor A related protein, high molecular weight carcinoembryonic antigen and nuclear matrix protein 22. With respect to the nuclear matrix protein 22, tests are available.

In addition, various studies have been conducted for identifying marker molecules for bladder cancer. For example, Lu Y., et.al., 2011, Am J Transl Res, 3(1), 8-27, describe that various genes are down-regulated in bladder cancer while other genes are up-regulated. For the ITIH5-gene, a down-regulation is described.

The bladder cancer is classified into different stages of disease based on location, size and spread of the cancer, according to the TNM (tumor, lymph node and metastasis) staging system. While stage 0 identifies that cancer cells are found only on the inner lining of the bladder, stage IV identifies that cancer cells have proliferated to the lymph nodes, pelvic or abdominal wall and/or other organs. In addition, recurrence of the cancer is possible. Stages 0 and 1 are identified as low grade cancer while stages 2 to 4 represent high grade cancer.

Various causes of treatment have been identified including surgery treatment as well as immunotherapy. For example, immunotherapy by BCG instillation is described to treat and prevent the recurrence of superficial tumors. In addition, radiation and other types of human therapy are envisaged for treating bladder cancer, in particular, bladder cancer of stages II, III and IV as well as recurring cancer.

The above referenced different stages of bladder cancer are roughly classified into two subtypes, namely, the superficial papillary carcinoma and the invasive high grade tumors. While the superficial (low grade) carcinoma has good prognosis of survival, patients suffering from high grade carcinoma or high grade tumors have a decreased survival rate and a higher degree of recurrence.

Until today, no reliable and solid method and means are available allowing diagnosis of bladder cancer by non-invasive analysis, in particular by analysis of urine-based samples.

Although the nuclear matrix protein 22 as well as other markers have been described as being useful in the diagnosis of bladder cancer, diagnostics based on the same are non-reliable, in particular, when applying non-invasive methods.

Recently, Vinci S., et.al., Urologic Oncology: Seminars and Original Investigations 2011, 29, 150-156, provides a quantative methylation analysis of BCL2, hTERT, and DAPK promoters in urine sediment. It is described that some of these genes may be a useful tool in the diagnosis of different types of urothelial carcinoma. Other markers have been described in the art. For example, WO2010/102823 relates to novel markers, namely FOXE1 and GATA4, for bladder cancer detection, in particular, for bladder cancer detection based on epigenetic changes. WO2009/069984 provides diagnosis kids and chips for bladder cancer using bladder cancer specific methylation marker genes CDX2, CYP1B1, VSX1, HOXA11, T, TBX5, PENK, PAQR9, LHX2, SIM2. In US2009/0054260 methylation levels of 9 markers in urine sediment are analysed for identifying bladder cancer, namely of the genes p16, ARF, GSTP1, MGMT, RAR-beta2, TIMP3, CDH1, RASSF1A, LOXL1, LOXL4 and APC. US2010/0317000 relates to a method for diagnosing bladder cancer by analyzing DNA methylation profiles in urine sediments and its kits. Therein, the following genes are identified: ABCC13, ABCC6, ABCC8, ALX4, APC, BCAR3, BCL2, BMP3B, BNIP3, BRCA1, BRCA2, CBR1, CBR3, CCNA1, CDH1, CDH13, CDKN1C, CFTR, COX2, DAPK1, DRG1, DRM, EDNRB, FADD, GALC, GSTP1, HNF3B, HPP1, HTERT, ICAM1, ITGA4, LAMA3, LITAF, MAGEA1, MDR1, MGMT, MINT1, MINT2, MT1GMT, MT1a, MTSS1, MYOD1, OCLN, p14ARF, p16INK4a, RASS1A, RPRM, RUNX3, SALL3, SERPINB5, SLC29A1, STAT1, TMS1, TNFRSF10A, TNFRSF10C, TNFRSF10D, TNFRSF21, and WWOX.

In addition, Costa V., et al., al. 2010, Clin Cancer Res, 16(23):5842-51, describe recently three new epigenetic biomarkers, GDF15, TMEFF2, and VIM which should predict bladder cancer from DNA-based analyses of urine samples. However, the *vimentin* gene (VIM) showed a higher frequency of methylation in DNA preparations from urine samples of healthy individuals in our analysis (see below). An object of the present invention is to provide methods allowing for diagnosis or identifying bladder cancer in a subject based on a minimal set of genes with high specificity and high sensitivity. Another object of the present invention is to provide a method for predicting a clinical outcome or determining the treatment course in a subject afflicted with bladder cancer. In addition, another aim of the present application is to provide methods allowing for the stratification of the therapeutic regimen of a subject suffering from bladder cancer as well as methods for allowing monitoring the progress of bladder cancer in the subject.

In particular, diagnostic tools and methods are required allowing differentiation between bladder cancer and other types of cancers, like prostate cancer.

The present invention aims for providing new methods as well as biomarkers and kits including the same particularly useful in the issues described above.

### Summary of the present invention

In a first aspect, the present invention relates to a method for diagnosing or identifying bladder cancer in a subject comprising
a) determining the level or amount of methylation of the promoter of the *ECRG4-* and/or *ITIH5*-gene, in particular, of the nucleic acid sequence of Seq. ID No. 1 and/or Seq. ID No. 2; in a sample of said subject; and
b) diagnosing or identifying bladder cancer based on the level or amount of methylation of the promoter of the ECRG4- and/or ITIH5-gene.

DNA methylation is the main epigenetic modification in humans. It is a chemical modification of DNA performed by enzymes called DNA methyltransferases, in which a methyl group (m) is added to specific cytosine (C) residues in DNA. In mammals, methylation occurs only at cytosine residues adjacent to a guanosine residue, i.e. at the sequence CG, also called the CpG dinucleotide. In normal cells, methylation occurs predominantly in regions of DNA that have few CG base repeats, while so-called CpG islands, regions of DNA that have long repeats of CG bases, remain non-methylated. Gene promoter regions that control gene transcription and thus protein expression are often CpG island-rich. Aberrant methylation of these normally non-methylated CpG islands in the promoter region causes transcriptional inactivation or silencing of important functional genes in human cancers, i.e. tumor suppressor genes.

In a further aspect, the present invention relates to a method for predicting a clinical outcome or determining the treatment course in a subject afflicted with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 in at least one sample of said subject; and
b) predicting the clinical outcome or determining the treatment cause based on the level or amount of methylation of the ECRG4- and/or ITIH5-gene.

Another embodiment of the present invention relates to a method for the stratification of the therapeutic regimen of a subject with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2; and
b) determining the therapeutic regimen of said subject based on the level or amount of methylation of the promoter of ECRG4- and/or ITIH5-gene.

Another embodiment of the present invention relates to a method for monitoring the progress of bladder cancer in a subject being diagnosed for bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 at a first time point; and, optionally,
b) determining the level or amount of methylation of the promoter of the ECRG4- and/or ITIH5-gene, in particular, of the nucleic acid sequence of Seq. ID No. 1 and/or Seq. ID No. 2 at a second time point; and, optionally,
c) comparing the level or amount of methylation determined in step a) to the level or amount detected in step b) or to a reference value.

That is, the present inventors recognised that the level or amount of methylation of the promoter of the ECRG4- and/or the promoter of the ITIH5-gene in a sample of the subject, in particular, in a urine sample, represents a suitable biomarker for diagnosing or identifying bladder cancer as well as determining the treatment course and predicting the clinical outcome including the stratification of a therapeutic regimen and monitoring the progress of bladder cancer.

Moreover, the present invention relates to a biomarker for bladder cancer which is at least one of the promoters of the ECRG4- and/or ITIH5-gene, in particular, the level or amount of methylation of said promoter of the respective genes.

Finally, the present invention provides a test kit for use in a method according to the present invention comprising means for determining the level or amount of methylation of the promoter of the ECRG4- and/or ITIH5-gene in a body fluid sample of a subject to be tested, in particular, of a urine sample, and instructions on how to use said test kit for a method according to the present invention.

It is preferred that said test kit is a test kit allowing pyro-sequencing or qMSP (quantitative methylation-specific PCR).

### Brief description of the drawings

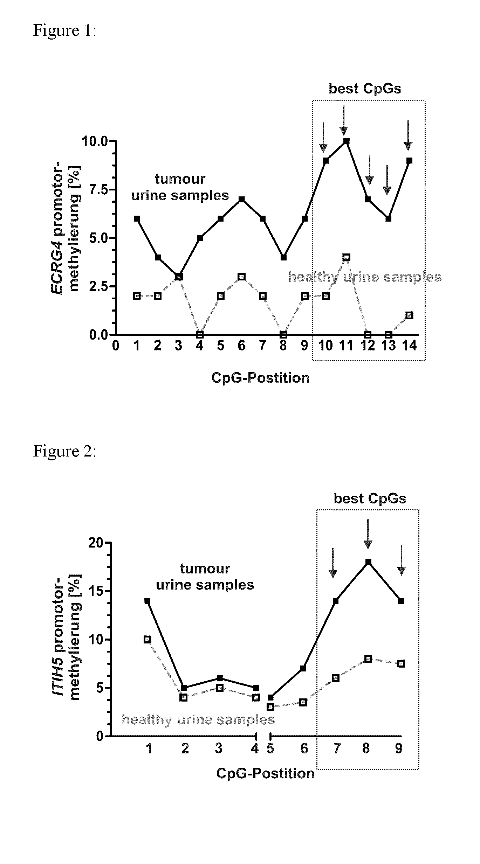

Figure 1: **Frequency of *ECRG4* methylation determined by pyrosequencing.** Median methylation rates for each CpG site of DNA preparations derived from urine samples of bladder cancer patients and healthy donors, respectively. Arrows indicate CpG sites with highest median difference in methlyation rate between tumour urine samples and healthy urine samples (control group).
Figure 2: **Frequency of ITIH5 methylation determined by pryose-quencing.** Median methylation rates for each CpG site of DNA preparations derived from urine samples of bladder cancer patients and healthy donors, respectively. Arrows indicate CpG sites with highest median difference in methlyation rate between tumour urine samples and healthy urine samples (control group).

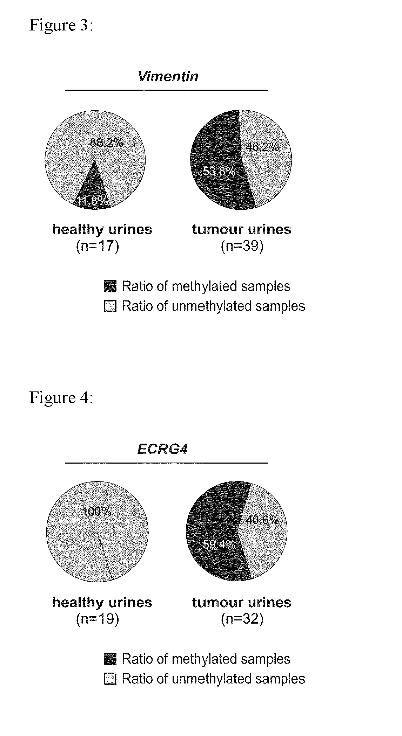

Figure 3: **Sensitivity and specificity of Vimentin methylation in DNA preparations derived from urine samples of bladder cancer patients and healthy donors, respectively**. *Left diagram*: Specificity. *Right diagram*: Sensitivity. Note, that Vimentin promoter methylation is also detected in DNA preparations derived from urine of healthy donors.
Figure 4: **Sensitivity and specificity of ECRG4 methylation in DNA preparations derived from urine samples of bladder cancer patients and healthy donors, respectively.** *Left diagram*: Specificity. *Right diagram*: Sensitivity. Note, that ECRG4 promoter methylation is not detectable in DNA preparations derived from urine of healthy donors.

### Detailed description of the present invention

In a first aspect, the present invention provides a method for diagnosing or identifying bladder cancer in a subject, comprising:
a) determining the level or amount of methylation of the promoter of the ECRG4- and/or ITIH5-gene, in particular, of the nucleic acid sequence of Seq. ID No. 1 and/or Seq. ID No. 2, in a sample of said subject; and
b) diagnosing or identifying bladder cancer based on the level or amount of methylation of the promoter of the ECRG4- and/or ITIH5-gene.

That is, the present inventors recognised that depending on the level or amount of methylation of the promoter of the ECRG4-gene and/or ITIH5-gene, in a body fluid sample of a subject suspected to suffer from bladder cancer, it is possible to diagnose or identify bladder cancer in said subject, preferably based on an elevated methylation level or amount of said biomarker relative to a reference value.

It has been recognised that elevated level or amounts of methylation of the promoter of ECRG4- and/or ITIH5-gene, in particular, of the nucleic acid sequence of SEQ ID No. 1 and/or SEQ ID No. 2 in a sample of a subject suspected to be affected from bladder cancer allows to diagnose for or identify the same, hence, determining the level or amount of methylation of said promoters represents a promising new biomarker allowing to diagnose or identify said disease, determine disease severity and response to treatment as well as allowing for monitoring the progression of therapy and the stratification of therapy regimen in patients suffering from bladder cancer. In this connection, diagnosis includes the determination of the stage and grade of bladder cancer, respectively.

By "gene" is meant not only the particular sequences found in the publicly available database entries, but also encompasses transcript and nucleotide variants of these sequences. The term may also encompass any gene which is taken from the family to which the named "gene" belongs with the proviso that methylation or another epigenetic modification of the "gene" is linked to bladder cancer.

In the context of the present invention, the term "body fluid sample" or "sample of the body fluid" is a biological sample isolated from the subject which can include without being limited thereto, whole blood, serum and plasma. Preferably, the body fluid is urine. However, test samples for diagnostic, prognostic, or personalised medicinal uses can be obtained also from surgical samples, such as biopsies or fine needle aspirates, from paraffin embedded tissues, from frozen tumor tissue samples, or from fresh tumor tissue samples, from a fresh or frozen body fluid.

A "subject" in the context of the present invention is preferably a mammal. The mammal can be a human, non-human primate, mouse, rat, dog, cat, horse or cow but are not limited to these examples. A subject can be male or female. A subject can be one who has been previously diagnosed with or identified as suffering from or having bladder cancer and, optionally, but need not have already undergone treatment for said disease or disorder. A subject can also be one who has been diagnosed with or identified as suffering from bladder cancer, but show improvements in the disease as a result of receiving one or more treatments for said disease or disorder. Moreover, a subject may also be one who has not been briefly diagnosed or identified as suffering from bladder cancer. A subject can also be one who is suffering from or at risk of developing bladder cancer, e.g., due to genetic predisposition.

The term "determining" as used herein refers to assessing the presence, absence, quantity, level or amount of either a given substance within a clinical or subject derived sample. In particular, the term "determining" refers to assess the level or amount of the methylation of a given DNA sequence.

"Bladder cancer" is defined to include transitional cell carcinoma or squamous cell carcinomas. The cancer may comprise superficial bladder cancer, invasive bladder cancer, or metastatic bladder cancer. Superficial cancer is only in cells in the lining of the bladder and has high grade of recurrence. A superficial tumor may grow through the lining into the muscular wall of the bladder and become invasive cancer. Invasive cancer can extend through the bladder wall and can grow into a nearby organ such as the uterus or vagina (in women) or the prostate gland (in men). It also may invade the wall of the abdomen. The cancer becomes metastatic when it spreads outside the bladder into nearby lymph nodes and other organs, such as the lungs, liver, or bones. Various stages of bladder cancer to which the invention is applicable are listed in the tables in the experimental section herein.

In a further aspect, the present invention relates to a method for predicting a clinical outcome or determining the treatment course in a subject afflicted with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 in at least one sample of said subject; and
b) predicting the clinical outcome or determining the treatment course based on the level or amount of methylation of the ECRG4- and/or ITIH5-gene.

That is, the person is in a position to identify the stage of the bladder cancer and, thus, can predict the development of the cancer and the treatment course. For example, when determining the level or amount of methylation at various time points, the artisan obtains information on the progress of the disease, the malignancy of the tumor etc.

Moreover, the present invention relates to a method for the stratification of the therapeutic regimen of a subject with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2; and
b) determining the therapeutic regimen of said subject based on the level or amount of methylation of the promoter of ECRG4- and/or ITIH5-gene.

Not to be bound to theory, it is submitted that depending on the level or amount of methylation, the stage of bladder cancer can be determined and, thus, the artisan is able to select an appropriate therapeutic regimen form the regimens known in the art.

In another embodiment, the present invention relates to a method for monitoring the progress of bladder cancer in a subject being diagnosed for bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 at a first time point; and, optionally,
b) determining the level or amount of methylation of the promoter of the ECRG4- and/or ITIH5-gene, in particular, of the nucleic acid sequence of Seq. ID No. 1 and/or Seq. ID No. 2 at a second time point; and, optionally,
c) comparing the level or amount of methylation determined in step a) to the level or amount detected in step b) or to a reference value.

In this connection, an increase of the level or amount of methylation is indicative for a progression or worsening of the disease.

It is preferred that both, the level or amount of methylation of the promoter of the ECRG4-gene and the level or amount of methylation of the promoter of the ITIH5-gene is determined in said sample.

It is preferred that the sample of the subject is a urine sample, in particular, the sediment of a urine sample.

Determination of the methylation status may be achieved through any suitable means. Suitable examples include bisulphite genomic sequencing and/or by methylation specific PCR. Various techniques for assessing methylation status are known in the art and can be used in conjunction with the present invention: sequencing, methylation-specific PCR (MS-PCR), melting curve methylation-specific PCR(McMS-PCR), MLPA with or without bisulphite treatment, QAMA, MSRE-PCR, MethyLight- or ConLight-MSP, bisulphite conversion-specific methylation-specific PCR (BS-MSP), COBRA (which relies upon use of restriction enzymes to reveal methylation dependent sequence differences in PCR products of sodium bisulphite - treated DNA), methylation-sensitive single-nucleotide primer extension conformation(MS-SNuPE), methylation-sensitive single-strand conformation analysis (MS-SSCA), Melting curve combined bisulphite restriction analysis, PyroMethA, HeavyMethyl, MALDI-TOF, MassARRAY, Quantitative analysis of methylated alleles (QAMA), enzymatic regional methylation assay (ERMA), QBSUPT, MethylQuant, Quantitative PCR sequencing and oligonucleotide-based microarray systems, Pyrosequencing, Next Generation Sequencing, Meth-DOP-PCR. A review of some useful techniques for DNA methylation analysis is provided e.g. in Nature Reviews, 2003, Vol.3, 253-266, which references are incorporated herein in their entirety. Techniques for assessing methylation status are based on distinct approaches. Some include use of endonucleases. Such endonucleases may either preferentially cleave methylated recognition sites relative to non-methylated recognition sites or preferentially cleave non-methylated relative to methylated recognition sites. Differences in cleavage pattern are indicative for the presence or absence of a methylated CpG dinucleotide. Cleavage patterns can be detected directly, or after a further reaction which creates products which are easily distinguishable. Means which detect altered size and/or charge can be used to detect modified products, including but not limited to electrophoresis, chromatography, and mass spectrometry.

Alternatively, the identification of methylated CpG dinucleotides may utilize the ability of the methyl binding domain (MBD) of the MeCP2 protein to selectively bind to methylated DNA sequences. The MBD may also be obtained from MBP, MBP2, MBP4, poly-MBD or from reagents such as antibodies binding to methylated nucleic acid. The MBD may be immobilized to a solid matrix and used for preparative column chromatography to isolate highly methylated DNA sequences. Variant forms such as expressed His-tagged methyl-CpG binding domain may be used to selectively bind to methylated DNA sequences. Eventually, restriction endonuclease digested genomic DNA is contacted with expressed His-tagged methyl-CpG binding domain. Other methods are well known in the art and include amongst others methylated-CpG island recovery assay (MIRA). Another method, MB-PCR, uses a recombinant, bivalent methyl-CpG-binding polypeptide immobilized on the walls of a PCR vessel to capture methylated DNA and the subsequent detection of bound methylated DNA by PCR.

Further approaches for detecting methylated CpG dinucleotide motifs use chemical reagents that selectively modify either the methylated or non-methylated form of CpG dinucleotide motifs. Suitable chemical reagents include hydrazine and bisulphite ions. The methods of the invention may use bisulphite ions, in certain embodiments. The bisulphite conversion relies on treatment of DNA samples with sodium bisulphite which converts unmethylated cytosine to uracil, while methylated cytosines are maintained. This conversion finally results in a change in the sequence of the original DNA. It is general knowledge that the resulting uracil has the base pairing behaviour of thymidine which differs from cytosine base pairing behaviour. This makes the discrimination between methylated and non-methylated cytosines possible. Useful conventional techniques of molecular biology and nucleic acid chemistry for assessing sequence differences are well known in the art and explained in textbooks.

Some techniques use primers for assessing the methylation status at CpG dinucleotides. Two approaches to primer design are possible. Firstly, primers may be designed that themselves do not cover any potential sites of DNA methylation. Sequence variations at sites of differential methylation are located between the two primers and visualisation of the sequence variation requires further assay steps. Such primers are used in bisulphite genomic sequencing, COBRA, Ms-SnuPE and several other techniques. Secondly, primers may be designed that hybridize specifically with either the methylated or unmethylated version of the initial treated sequence. After hybridization, an amplification reaction can be performed and amplification products assayed using any detection system known in the art. The presence of an amplification product indicates that a sample hybridized to the primer. The specificity of the primer indicates whether the DNA had been modified or not, which in turn indicates whether the DNA had been methylated or not. If there is a sufficient region of complementarity, e.g., 12, 15, 18, or 20 nucleotides, to the target, then the primer may also contain additional nucleotide residues that do not interfere with hybridization but may be useful for other manipulations. Examples of such other residues may be sites for restriction endonuclease cleavage, for ligand binding or for factor binding or linkers or repeats. The oligonucleotide primers may or may not be such that they are specific for modified methylated residues.

A further way to distinguish between modified and unmodified nucleic acid is to use oligonucleotide probes. Such probes may hybridize directly to modified nucleic acid or to further products of modified nucleic acid, such as products obtained by amplification. Probe-based assays exploit the oligonucleotide hybridisation to specific sequences and subsequent detection of the hybrid. There may also be further purification steps before the amplification product is detected e.g. a precipitation step. Oligonucleotide probes may be labelled using any detection system known in the art. These include but are not limited to fluorescent moieties, radioisotope labelled moieties, bioluminescent moieties, luminescent moieties, chemiluminescent moieties, enzymes, substrates, receptors, or ligands.

In the MSP approach, DNA may be amplified using primer pairs designed to distinguish methylated from unmethylated DNA by taking advantage of sequence differences as a result of sodium-bisulphite treatment (WO 97/46705). For example, bisulphite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulphite-modified DNA, whereas an oligonucleotide primer containing the guanine bases would hybridize to the non-modified (methylated) cytosine residues in the DNA. Amplification using a DNA polymerase and a second primer yield amplification products which can be readily observed, which in turn indicates whether the DNA had been methylated or not. Whereas PCR is a preferred amplification method, variants on this basic technique such as nested PCR and multiplex PCR are also included within the scope of the invention.

Suitable Primers and Probes for use in a method according to the present invention, in particular, for use in a PCR, are set forth in Seq. ID. Nos. 3 to 35. In particular, Seq. ID Nos. 3 to 5 identify a set of primers and probe for pyrosequencing of the ITIH5 gene, preferred, Seq. ID Nos. 6 to 8 are used for the pyrosequencing of the ITIH5 gene, to measure methylation of the preferred CpGs 7, 8 and 9 (best CpGs) as identified in table 2 below. For pyrosequencing of the ECRG4 gene, suitable nucleotides are set forth in Seq. ID Nos. 9 to 11.

For qMSP suitable primer pairs including probes are Seq. ID Nos. 21 to 23, 24 to 26, 27 to 29, 30 to 32, and 33 to 35 for ECRG4 and Seq ID Nos. 12 to 14, 15 to 17, and 18 to 20, respectively for ITIH5.

That is, the sample, in particular, the urine sample, and, preferably, the sediment of an urine sample, is obtained and DNA is isolated thereof. The level or amount of methylation is preferably determined based on the bisulfite method. That is, the DNA is treated with bisulfite before determining its pattern of methylation. In animals methylation is predominately in the addition of a methyl group to the carbon 5-postion of cytosine residues of the dinucleotide CpG, and it is implicated in repression of transcriptional activity. In the human DNA, typically 3% of cytosine (C) is methylated. Further, most cytosines are methylated in CpG dinucleotides. Methylation is involved in silencing gene expression.

Treatment of DNA with bisulfite converts cytosine residues to uracil but leaves 5'-methyl cytosine residues unaffected. Thus, by bisulfite treatment specific changes in the DNA sequence depending on the methylation status of individual cytosine residues can be introduced. Due to the individual changes in cytosine residues, it is possible to obtain single nucleotide resolution information about the methylation status of a sequence of DNA.

The converted DNA, i.e. the DNA obtained after bisulfite treatment, is then analysed for the presence of methylated cytosine. The analysis can be based on sequencing methods or on PCR-based methods or combinations thereof. Basically, it is possible to allocate the approaches for analysis into two classes, namely the non-methylation specific PCR-based methods and the methylation specific PCR also called MSP.

That is, it is possible determining the level or amount of methylation according to the present invention by different methods including the following: Direct sequencing, pyrosequencing, methylation sensitive single strand confirmation analysis, high resolution melting analysis, methylation sensitive single nucleotide primer extension or base specific cleavage using MALDI-TOF analysis.

Further, methylation specific PCR (MSP) may be applied, in particular, quantitative MSP analysis. Moreover, micro arraying based methods may be used.

It is particularly preferred that the determination is based on pyro-sequencing or qMSP (quantitative methylation specific PCR). The skilled person is well aware of said methods and conducting the same for determining the level or amount of methylation of the specified genes.

For qMSP suitable primer pairs including probes are Seq. ID Nos. 21 to 23, 24 to 26, 27 to 29, 30 to 32, and 33 to 35 for the ECRG4 gene and Seq ID Nos. 12 to 14, 15 to 17, and 18 to 20, respectively for the ITIH5 gene.

A specific example of the MSP technique is designated real-time quantitative MSP (qMSP), and permits reliable quantification of methylated DNA in real time or at end point. Real-time methods are generally based on the continuous optical monitoring of an amplification procedure and utilise fluorescently labelled reagents whose incorporation in a product can be quantified and whose quantification is indicative of copy number of that sequence in the template. One such reagent is a fluorescent dye, called SYBR Green I that preferentially binds double-stranded DNA and whose fluorescence is greatly enhanced by binding of double-stranded DNA. Alternatively, labelled primers and/or labelled probes can be used for quantification. They represent a specific application of the well known and commercially available real-time amplification techniques such as TAQMAN(R), MOLECULAR BEACONS(R), AMPLIFLUOR(R) and SCORPION(R), DzyNA(R), PlexorTM etc. In the real-time PCR systems, it is possible to monitor the PCR reaction during the exponential phase where the first significant increase in the amount of PCR product correlates to the initial amount of target template.

The present inventors recognised that high levels or amounts of methylation, enables to diagnose or identify bladder cancer. In addition, it has been identified that invasive high grade bladder cancer or the development of relapse of bladder cancer is associated with higher levels or amounts of methylation of the promoter of the ECRG4-gene and/or of the promoter of the ITIH5-gene, respectively.

In the context of the present invention, the term "reference value" refers to an index value, or a value derived from one or more bladder cancer risk prediction algorithms or computed indices, a value derived from a subject with the same disease or disorder, or a value derived from the subject diagnosed with or identified as suffering from bladder cancer. In particular, e.g. in case of the method for diagnosing or identifying bladder cancer, the reference value is obtained from subjects not afflicted with bladder cancer and, in addition, the reference value represents a range or index obtained from at least two samples collected from subjects not afflicted with bladder cancer.

The increase in the level or amount of methylation of the promoter of ECRG4-gene and/or ITIH5-gene is for example at least 10%, at least 15%, at least 20%, at least 25% or at least 50% of the reference value or normal control level, preferably, the increase is at least 100%. For example, the increase is at least twofold, threefold, fourfold or more.

Preferred embodiments and methods are applied allowing determining the methylation of single CpG present in the promoter of the ECRG4-gene and/or ITIH5-gene. For example, by applying pyro-sequencing or MSP, in particular, qMSP, it is possible to determine the level or amount of methylation of single CpG dinucleotides present in said promoter regions.

The promoter region of the ECRG4-gene comprises 14 CpG dinucleotides, suitable for early bladder cancer detection, according to the present invention while the promoter region of the ITIH5-gene contains 9 CpG dinucleotides, useful for early bladder cancer detection, according to the present invention. It is preferred that specific CpGs are analysed. For example, for the ECRG4-promoter it is preferred to analyse the level or amount of methylation of CpG10, CpG11, CpG12, CpG13, CpG14. For the ITIH5-promoter, preferred CpGs are CpG7, CpG8, and CpG9. In figure 1 and 2 as well as in tables 1 and 2, the positions of the CpG dinucleotides are shown.

**Table 1:**

| | **ECRG4 gene** |
|---|---|
| | **Nucleotide position (5'-3')^{a}** |
| **CpG Number** | |
| **1** | 337-338 |
| **2** | 347-348 |
| **3** | 351-352 |
| **4** | 355-356 |
| **5** | 362-363 |
| **6** | 368-369 |
| **7** | 390-391 |
| **8** | 392-393 |
| **9** | 395-396 |
| **10** | 403-404 |
| **11** | 413-414 |
| **12** | 418-419 |
| **13** | 424-425 |
| **14** | 427-428 |

| | |
|---|---|
| **^{a}Position is related to the ECRG4 gene of Seq. ID No. 1 (5'-3')** | |

**Table 2:**

| | **ITIH5 gene** |
|---|---|
| | **Nucleotide position (5'-3')^{a}** |
| **CpG Number** | |
| **1** | 487-488 |
| **2** | 489-490 |
| **3** | 499-500 |
| **4** | 519-520 |
| **5** | 664-665 |
| **6** | 692-693 |
| **7** | 723-724 |
| **8** | 725-726 |
| **9** | 728-729 |

| | |
|---|---|
| **^{a}Position is related to the ITIH5 gene Seq. ID No. 2 (5'-3')** | |

It is preferred that the methylation of single or individually combined CpG sites is determined by pyro-sequencing or qMSP.

It is preferred that the level of a methylation of the CpGs is above 5% in average of the DNA analysed. In particular, it is preferred that the level of methylation as determined by pyrosequencing or qMSP is above 5, like 6, 7, 8, 9, and preferably above 10%, in particular, of CpG 10, 11, 12, 13 and 14 of the ECRG4-promoter region as well as at least above 10% for CpG 7,8 and 9 of the ITIH5-promoter region. Furthermore, it is preferred that the absolute amount of the difference in methylation is at least 5, like at least 6 when comparing the tumour sample with a healthy donor sample.

It is particular preferred that the level of methylation is at least threefold, like fourfold higher in subjects afflicted or deemed to be afflicted with bladder cancer compared to a subject not afflicted.

The methods according to the present invention are suitable for allowing differentiation between bladder cancer and prostate cancer. That is, while according to the present invention, the level or amount of methylation of the promoter regions defined therein is higher in bladder cancer patients, patients suffering from prostate cancer do not have elevated levels or amounts of methylation of said promoter genes.

Suitable controls may need to be incorporated in order to ensure the method chosen is working correctly and reliably. Suitable controls may include assessing the methylation status of a gene known to be methylated. This experiment acts as a positive control to help to ensure that false negative results are not obtained. The gene may be one which is known to be methylated in the sample under investigation or it may have been artificially methylated, for example by using a suitable methyltransferase enzyme, such as Sssl methyltransferase. In one embodiment, the gene selected from ECRG4 and ITIH5, may be assessed in normal (i.e. non-cancerous bladder) cells, following treatment with Sssl methyltransferase, as a positive control.

Additionally or alternatively, suitable negative controls may be employed with the methods of the invention. Here, suitable controls may include assessing the methylation status of a gene known to be unmethylated or a gene that has been artificially demethylated. This experiment acts as a negative control to ensure that false positive results are not obtained. In one embodiment, the gene selected from ECRG4 and ITIH5 may be assessed in normal (bladder) cells as a negative control, since it has been shown for the first time herein that these genes are unmethylated in normal (bladder) tissues.

All methods of the present invention may be used in connection with bladder cancer. To attain high rates of tumor detection, it may be advantageous to complement the methods of the invention with established methods for bladder cancer identification. Non-invasive methods may be especially suitable for use in combination with the noninvasive methods of the invention. Methods of the present invention may be used in conjunction with one or more of the following methods: - Urinalysis Urine cytology (microscopic exam of urine to look for cancerous cells)
- Cystoscopy (use of lighted instrument to view inside bladder. Diagnosis and staging of bladder cancer begins with cystoscopy)
- Bladder biopsy (usually performed during cystoscopy) - Intravenous pyelogram - IVP (Dyes are injected into the bloodstream, which allow for better visualization of any tumors or abnormalities in the bladder using routine X- rays.)
- Imaging Techniques: X-ray imaging of the upper urinary tract (including the ureters and kidneys) may be done to rule out any involvement of these structures. Ultrasound can be used to study the kidneys and a CT scan is often very good at studying the entire length of the urinary tract.

More recently, urine-based marker tests are being developed and provide yet another means to complement the methods of the invention. These new tests are non-invasive and accurate in detecting low-grade bladder cancer and therefore are especially useful in monitoring for recurrence, including BTA assays detecting hCFHrp, or human complement factor H-related protein, which is present in the urine of patients with bladder cancer. There are both quantitative and qualitative BTA methods available; NMP22 Test Kit detecting a nuclear mitotic apparatus (NMA) protein that is abundant in the nuclear matrix. In bladder tumor cells, NMA is elevated and released in detectable levels. There are both quantitative and qualitative NMP22 methods; the Vysis UroVysion assay combining urine cytology with molecular (DNA-based) technology to detect the recurrence of cancer. It employs Fluorescence in situ Hybridization (FISH) technology, which uses small, fluorescently-labelled DNA probes to microscopically identify specific regions of DNA; ImmunoCyt being an immunocytochemistry assay for the detection of Mucin and CEA antigens expressed by tumor cells in the urine of patients previously diagnosed with bladder cancer. This immunofluoresence method is to be combined with urine cytology for the early detection of bladder cancer recurrence. ImmunoCyt is a qualitative assay.

It is preferred that the analysis of the values obtained after determining the amount or level of methylation is analysed by ROC (receiver operating characteristic). Based on said ROC analysis, it is possible to identify a cut of level of sensitivity above 80 or even above 85% while having a specificity of above 90%, e.g. of having 100% specificity. That is, it is possible to have 100% specificity while sensitivity is above 80% based on ROC analysis. This is particularly true for the preferred embodiment of the analysis of both, the promoter of the ECRG4-gene and the ITIH5-gene.

It is noteworthy that for the methods according to the present invention only small amounts of the sample are required and, in addition, that the methods do not necessitate any surgery steps when using urine as biological sample. In addition, it is possible to define cut off levels, thus, providing suitable methods for diagnosis and stratification as well as determining the treatment course of bladder cancer. Hence, the methods according to the present invention are particularly valuable for medical prevention of cancer as well as for risk assessment of in subjects suffering from bladder cancer.

One aspect of the present invention relates to a method for allowing stratification of therapeutic regimen of said subject afflicted with bladder cancer based on determining the level or amount of methylation of the promoter of the ECRG4-gene and/or the promoter of the ITIH5-gene, in particular, the nucleic acid sequences of SEQ ID No. 1 and/or SEQ ID No. 2 and determining the therapeutic regimen based on the level or amount of said methylation. That is, by determining the level or amount of methylation especially in the urine of said subject, allows the attending position to determine and predict the usefulness of therapy based on conventional therapeutics for said disease.

In a further aspect, the present invention relates to the use of an ECRG4-gene and/or the ITIH5-gene, in particular, of the promoter of the ECRG4-gene or the promoter of the ITIH5-gene as a biomarker for bladder cancer. In particular, the level or amount of methylation of said promoters are suitable as a biomarker for bladder cancer.

Thus, a kit is provided for detecting a predisposition to, or the incidence of, bladder cancer in a sample (the sample comprising nucleic acid molecules from bladder cells, as defined herein) comprising at least one primer pair and/or probe for determining the methylation status of each gene in a panel of genes wherein the panel of genes comprises, consists essentially of or consists of a panel of genes selected from ECRG4 and ITIH5. As discussed herein, these genes have been shown to be useful in predicting or diagnosing bladder cancer, non-invasively, with excellent sensitivity and specificity. Suitable primer pairs for determining the methylation status of each of the genes of the panel are set forth in the examples below. The primers and/or probe may permit direct determination of the methylation status of the panel of genes, for example following bisulphite treatment of the DNA. Thus, they may be MSP or bisulphite sequencing primers for example. The kits may additionally include one or more probes for real-time or end-point detection. The probes may additionally or alternatively permit direct determination of the methylation status of the panel of genes, for example following bisulphite treatment of the DNA. Blocking probes may also be utilised in certain embodiments, according to the Heavymethyl technique.

Suitable Primers and Probes for use in a kit according to the present invention, in particular, for use in a PCR, are set forth in Seq. ID. Nos. 3 to 35. In particular, Seq. ID Nos. 3 to 5 identify a set of primers and probe for pyrosequencing of the ITIH5 gene, preferred, Seq. ID Nos. 6 to 8 are used for the pyrosequencing of the ITIH5 gene, to measure methylation of the preferred CpGs 7, 8 and 9 (best CpGs) as identified in table 2. For pyrosequencing of the ECRG4 gene, suitable nucleotides are set forth in Seq. ID Nos. 9 to 11.

For qMSP suitable primer pairs including probes are Seq. ID Nos. 21 to 23, 24 to 26, 27 to 29, 30 to 32, and 33 to 35 for ECRG4 and Seq ID Nos. 12 to 14, 15 to 17, and 18 to 20 for ITIH5, respectively.

The primers and/or probe may investigate the methylation status of the relevant gene or genes. In certain embodiments, the primers and/or probe may investigate the methylation status within, or between, and optionally including, the primer and/or probe binding sites of the primers and/or probes listed in the examples. In specific embodiments, the primers and/or probes may investigate the methylation status, within or between the genomic locations identified in the examples and in figures. Thus, for example, the primers and/or probes may investigate the genomic region between (and including) nucleotide 330 and nucleotide 440 for ECRG4 according to Seq. ID No.1 and/or the genomic region between (and including) nucleotide 480 and nucleotide 740 for ITIH5 according to Seq. ID No.2. Preferably, the primer and probes are primer pairs and corresponding probes as defined above.

The kit may further comprise means for processing a urine sample (containing bladder cells or genomic DNA from bladder cells). The kit may further comprise:
(a) means for detecting methylation in at least one gene selected from ECRG4 and ITIH5
(b) means for processing a urine sample.

The means for detecting the methylation status may permit the methylation status to be identified directly, for example the means may comprise primers and/or probes that investigate the methylation status directly (e.g. MSP primers or Heavymethyl probes).

The kits may enable the detection to be carried out in a single reaction, for example by including suitably labelled primers or probes or by selecting amplification products which can be readily distinguished according to size, molecular weight etc.

The kit may be for use in MSP and may enable a real-time detection version of MSP. In some embodiments the kit permits an end-point detection version of MSP to be carried out. Thus, the means for detecting an epigenetic change may comprise, consist essentially of or consist of suitable primers for determining whether the at least one gene selected from ECRG4 and ITIH5 (together, optionally, with additional genes) is methylated. These primers may comprise any of the primers discussed in detail in respect of the various methods of the invention which may be employed in order to determine the methylation status of the relevant (at least one) gene, and variants thereof. The kit may further comprise probes for real-time detection of amplification products. The probes may comprise any suitable probe type for real-time detection; non-limiting examples include use of TAQMAN probes and/or MOLECULAR BEACONS probes and/or AMPLIFLUOR primers and/or FRET probes and/or SCORPION primers and/or oligonucleotide blockers. Such kits for real-time detection may also be used for end-point detection. Suitable probes and primer are oligonucleotides according to Seq ID. Nos. 12-35.

The primers and/or probes may permit direct determination of the methylation status of the at least one gene in all of the kits of the invention, for example following bisulphite treatment of the (DNA in the) sample, as discussed herein.

Suitable Primers and Probes according to the present invention, in particular, for use in a PCR, are set forth in Seq. ID. Nos. 3 to 35. In particular, Seq. ID Nos. 3 to 5 identify a set of primers and probe for pyrosequencing of ITIH5, preferred, Seq. ID Nos. 6 to 8 are used for the pyrosequencing of ITIH5, to measure methylation of the preferred CpGs 7, 8 and 9 (best CpGs) as identified in table 2. For pyrosequencing of the ECRG4 gene, suitable nucleotides are set forth in Seq. ID Nos. 9 to 11.

For qMSP suitable primer pairs including probes are Seq. ID Nos. 21 to 23, 24 to 26, 27 to 29, 30 to 32, and 33 to 35 for ECRG4 and Seq ID Nos. 12 to 14, 15 to 17, and 18 to 20 for ITIH5, respectively.

The primers and/or probes may be labelled as required. FAM and DABCYL are representative examples of fluorescent markers which can participate in FRET to provide a reliable indicator of amplification, as discussed herein. Other fluorophores and quenchers may be employed, in particular as FRET pairs, as desired and as would be appreciated by a skilled person.

The primers and/or probe may investigate the methylation status, of the relevant gene or genes. In certain embodiments, the primers and/or probe may investigate the methylation status within, or between, and optionally including, the primer and/or probe binding sites of the primers and/or probes listed in the table.

As indicated herein above, the kit may comprise means for processing a urine sample. Such means for processing a urine sample may comprise a stabilising buffer in certain embodiments. Suitable stabilising buffers are described herein and may incorporate appropriate mixtures of buffering and osmolarity adjustment ingredients. Examples include STABILUR tablets, available from Cargille Labs and preservative tubes available from CellSave (CellSave Preservative Tubes).

The kit may further incorporate reagents for extraction / isolation / concentration/purification of DNA in certain embodiments. In further embodiments, the kit may also incorporate a sealable vessel for collection of a urine sample. In certain embodiments, the kit of the invention further comprises a reagent which modifies unmethylated cytosine (but not methylated cytosine) or vice versa in detectable fashion. This allows methylated residues to be distinguished from non-methylated residues. In certain embodiments, the reagent converts unmethylated cytosine residues to a different nucleotide (uracil) but methylated residues are not converted. In certain embodiments, the reagent comprises bisulphite, preferably sodium bisulphite but may comprise hydrazine for example.

As discussed, suitable controls may be utilised in order to act as quality control for the methods and be included in the kit of the invention. One example of a suitable internal reference gene, which is generally unmethylated, but may be treated so as to be methylated, is [beta]-actin. The kit of the invention may further comprise primers for the amplification of a control nucleic acid which may comprise at least one gene selected from ECRG4 and ITIH5 in unmethylated and/or methylated form.

The kits of the invention may additionally include suitable buffers and other reagents for carrying out the claimed methods of the invention. In certain embodiments, the kit of the invention further comprises, consists essentially of, or consists of nucleic acid amplification buffers.

The kit may also additionally comprise, consist essentially of or consist of enzymes to catalyze nucleic acid amplification. Thus, the kit may also additionally comprise, consist essentially of or consist of a suitable polymerase for nucleic acid amplification. Examples include those from both family A and family B type polymerases, such as Taq, Pfu, Vent etc.

The various components of the kit may be packaged separately in separate compartments or may, for example be stored together where appropriate. The kit may also incorporate suitable instructions for use, which may be printed on a separate sheet or incorporated into the kit packaging for example.

That is, in another aspect the present invention relates to kit for use in a method according to the present invention for predicting the clinical outcome or determining the treatment course in a subject, for diagnosing or identifying bladder cancer in a subject, for the stratification of the therapeutic regimen, or for monitoring the progression of bladder cancer in a subject supposed to have or afflicted with bladder cancer, said kit comprises a means for determining the level or amount of methylation of the promoter of the ECRG4- and/or ITIH5-gene, in particular, of the nucleic acid sequence of SEQ ID No. 1 and/or SEQ ID No. 2 in a body fluid sample of a subject to be tested, in particular, of a urine sample, and instructions on how to use said test kit for a method according to the present invention.

It is particularly preferred that said kit allows pyro-sequencing or qMSP of the biological sample, in particular, DNA obtained from a human sample of said subject.

Generally, it is preferred that in the method according to the present invention as well as in a kit according to the present invention both, ECRG4 gene and ITIH5 gene are analysed for methylation. Furthermore, it is preferred that both, the promoter of the ECRG4-gene and the ITIH5 gene, in particular, the level or amount of methylation thereof, are used as a biomarker for bladder cancer.

The above disclosure generally describes the present invention. A more complete understanding can be obtained by reference to the following specific examples of the embodiments of the invention without being limited thereto.

### Methods

### Patients and design study

Urine samples were obtained from the University Hospital Aachen. Only, urine samples from patients with bladder cancer, prostate cancer and inflammatory diseases (cystitis) were used. Patients with more than one cancer type (e.g. bladder and prostate cancer) were excluded. According to the relevant demographic data urine samples from healthy donors were age matched and used for controls. All patients gave informed consent for retention and analysis of their urine samples for research purposes, and the Ethics Committees of the respective centres approved the study.

### Urine sample preparation

Morning voided urine samples were collected. The storage and processing conditions of urine samples were standardised: 20 ml of urine samples were filled in a 50ml falcon tube and centrifuged for 10 minutes at 1500 x g. Afterwards cell sediments were transferred into a cryo-tube and stored at -80°C.

### DNA isolation from urine

Cryo-conserved urine sediments were gently defrosted on ice and then resuspended in ddH₂O. Subsequently, the DNA was isolated using the ZR Urine DNA Isolation Kit^{™} according to the manufacturer's instructions (Zymo Reserach, Freinburg i. Breisgau, Germany).

### Bisulfite-modification

Bisulfite treatment of DNA was performed as previously described (Veeck J., et al., 2008, Oncogene, 27(6):865-76).

### Pyrosequencing

Based on the specific ECRG4 and ITIH5 assays e.g. using the primer set according to Seq ID Nos. 3 to 5, 6 to 8, and 9 to 11, respectively, for determining methylation of CpG's shown in tables 1 and 2, respectively, pyrosequencing analysis was performed as previously described (Noetzel E., et al., 2010, Oncogene, 29(34):4814-25).

### Real-time quantitative metyhlation-specific PCR (qMSP)

Primers and probes for qMSP assays of the ITIH5-gene and the ECRG4-gene of Seq. ID. Nos. 12 to 14, 15 to 17, 18 to 20, 21 to 23, 24 to 26, 27 to 29, 30 to 32, and 33 to 35 for determining methylation of CpG's shown in tables 1 and 2, respectively were designed to bind to bisulfite converted DNA using Beacon Designer Software (Premierbiosoft). Bis-DNA amplification was carried out in triplicates and performed in an iCycler IQ5 (Bio-Rad Laboratories, Munich, Germany).

Results: Initial DNA methylation analysis of the *ITIH5-* and *ECRG4*-gene promoter identified distinct regions with a high biomarker potential, respectively (Figure 1 and 2). Analayzing ITIH5 and ECRG4 gene promoter methylation in urine samples of bladder cancer patients,frequent methylation was detected achieving an overall-panel sensitivity (ITIH5 and ECRG4) of approximately 80%. None of the age-matched control urine samples exhibited methylation signals. We compared the ITIH5 and ECRG4 gene methylation to methylation of the Vimentin (VIM) gene, that was recently published as a putative DNA methylation biomarker (Costa VL., et al. 2010, Clin Cancer Res, 16(23):5842-51). When Vimentin was tested in the same control cohort, it exhibited an increased false-positive rate (Figure 3 und 4). Subsequently, pyrosequencing was used to determine the frequency of ITIH5 and ECRG4 gene methylation in a "validation cohort" of urine samples. This confirmed the biomarker potential of both the ITIH5 and ECRG4 gene. Biomarker performances of ITIH5 and ECRG4 promoter regions were further optimized by using receiver operator characteristics (ROC) curve analysis. Of importance, this analysis provided CpG sites (named "best CpGs" in Figures 1 and 2) with best biomarker performance to discriminate between urine samples from bladder cancer patients and healthy individuals. Combining the DNA methylation biomarkers ITIH5 and ECRG4, an overall-two-gene panel sensitivity of 83.9% (p<0.001, AUC=0.916) with 100% specificity was achieved as shown in table 3. In the clinical important group of pTaG1 *low grade* TCC tumours the panel sensitivity was comparable high, showing 88.9% sensitivity and 100% specificity (based on the cut-off-level of ≥4.65).

**Table 3:**

| | **Gene promoter loci with biomarker potential** | | | | | non-suitable gene locus |
|---|---|---|---|---|---|---|
| | **ECRG4** | **ECRG4 (Best CpG)** | **ITIH5** | **ITIH5 (Best CpG)** | **ECRG4** + **ITIH5 Panel** | **ECRG4_downstrea m** |
| **Cut-Off-Level^{a}** | ≥4.75 | ≥4.5 | ≥7.5 | ≥15 | ≥4.65 | ≥6.5 |
| **Sensitivity [%]** | 63.4 | 65.9 | 51.6 | 51.7 | 83.9 | 45.2 |
| **Specificity [%]** | 100 | 100 | 100 | 100 | 100 | 100 |
| **AUC^{b}** | 0.895 | 0.909 | 0.795 | 0,754 | 0.916 | 0.668 |
| **P-value^{c}** | <0.001 | <0.001 | 0.002 | 0.008 | <0.001 | 0.07 (*not significant)* |

| | | | | | | |
|---|---|---|---|---|---|---|
| ^{a}Cut-Off-Level condition: 100% specificty. ^{b}AUC = Area Under Curve (ROC analysis-based). ^{c}Asymptotic significance level of 0.05 (95% AUC confidence intervals) | | | | | | |

Conclusion:

Two novel biomarker, i.e. ITIH5 and ECRG4 gene promoter methylation, have been identified. Determing ITIH5 and ECRG4 promoter methylation in urine sediments (e.g. using pyrosequencing or qMSP) is suitable to detect primary bladder cancer with very high sensitivity and specificity. Of special clinical importance, frequently recurring pTaG1 bladder tumours were detected with comparable high sensitivity and specificity. Targeting the best CpG sites could improve the preferred 2-gene-panel biomarker performance, thus opens up a non-invasive alternative approach for early bladder cancer diagnosis compared to current clinical standards as well as to recently published DNA methylation biomarkers candidates in bladder cancer such as the Vimentin gene.

## Claims

1. A method for diagnosing or identifying bladder cancer in a subject, comprising:
a) determining the level or amount of methylation of the promoter of the ECRG4- and/or ITIH5-gene, in particular, of the nucleic acid sequence of Seq. ID No. 1 and/or Seq. ID No. 2, in a sample of said subject; and
b) diagnosing or identifying bladder cancer based on the level or amount of methylation of the promoter of the ECRG4- and/or ITIH5-gene.

2. A method for predicting a clinical outcome or determining the treatment course in a subject afflicted with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 in at least one sample of said subject; and
b) predicting the clinical outcome or determining the treatment course based on the level or amount of methylation of the promoter of the ECRG4- and/or ITIH5-gene.

3. A method for the stratification of the therapeutic regimen of a subject with bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2; and
b) determining the therapeutic regimen of said subject based on the level or amount of methylation of the promoter of ECRG4- and/or ITIH5-gene.

4. A method for monitoring the progress of bladder cancer in a subject being diagnosed for bladder cancer, comprising:
a) determining the level or amount of methylation of the promoter of the ECRG4- and/or ITIH5-gene, in particular, of the nucleic acid sequence of Seq. ID No. 1 and/or Seq. ID No. 2 at a first time point; and, optionally,
b) determining the level or amount of methylation of the promoter of the ECRG4- and/or ITIH5-gene, in particular, of the nucleic acid sequence of Seq. ID No. 1 and/or Seq. ID No. 2 at a second time point; and, optionally,
c) comparing the level or amount of methylation determined in step a) to the level or amount detected in step b) or to a reference value.

5. The method of any one of the preceding claims where a high level or amount of methylation is regarded as an indicator for bladder cancer, for invasive high grade bladder cancer or for the development of relapse of bladder cancer, respectively.

6. The method according to any one of the preceding claims wherein the sample of the subject is a urine sample, in particular, the sediment of a urine sample after centrifugation.

7. The method according to claim 2 or 4 wherein the first sample is taken from the subject prior treatment for bladder cancer and/or the second sample is taken from the subject after being treated for bladder cancer.

8. The method according to any one of the preceding claims wherein determining methylation of the promoter of the ECRG4- and/or ITIH5-gene comprises determining of methylation of single CpG.

9. The method according to claim 8 wherein determining the methylation of said promoter includes determining the level or amount of methylation of the CpGs 1 to 9 of the ITIH5-promoter and/or 1 to 14 of the ECRG4-promoter according to table 1 and 2, respectively.

10. A method according to any one of the preceding claims **characterised in that** the methylation is determined by pyro-sequencing or qMSP.

11. A method according to any one of the preceding claims wherein at least the level of methylation of at least one of the CpG 10-14 of the ECRG4-promoter and/or at least one of the CpGs 7-9 of the ITIH5-promoter according to table 1 and 2, respectively, is determined.

12. The method according to any one of the preceding claims allowing differentiation between bladder cancer and prostate cancer.

13. The use of a kit in predicting a clinical outcome or determining the treatment cause, for diagnosing or identifying, for the stratification of the therapeutic regimen, or for monitoring the progression of bladder cancer in a subject supposed to have or afflicted with bladder cancer, said kit comprises a means for determining the level or amount of methylation of the promoter of the ECRG4- and/or ITIH5-gene, in particular, of the nucleic acid sequence of SEQ ID No. 1 and/or SEQ ID No. 2 in a body fluid sample of a subject to be tested, in particular, of a urine sample, and instructions on how to use said test kit for a method according to any one of claims 1 to 12 .

14. The use of a kit acquired to claim 13 whereby said kit is a kit for pyro-sequencing or qMSP.

15. The use of at least one of the promoter of the ECRG4-gene or the promoter of the ITIH5-gene, in particular, the level or amount of methylation thereof, as a biomarker for bladder cancer.


SEQUENCE LISTING

<110>  RWTH Aachen

<120>  Biomarker for bladder cancer

<130>  4589-008 EP

<160>  35

<170>  PatentIn version 3.5

<210>  1
<211>  1207
<212>  DNA
<213>  human

<400>  1
aagcagggag ggagggagat agggaagaaa ggggtgagga gtaaggggaa cagtggcgcg      60

gctggggcgg gcggaggaag tgggggagcc aaggagacac cccagcgctg ggatccggca     120

agtcctccct ctgagtggcc agggggcctc gtcccttctc ccgatgcctt ctgcccttcc     180

ttgggtctcc ggaacccagc ttgtcctaac cgctttcgct gcgggcagcg ctggccacgc     240

ggcccccgcc gccggcggtt ctccgtggcc aagcatcctt ggccttggag cccaggggct     300

gcgttcccct tggggccggg gcgggagaga ggacctcggt ggtactcgcc cgtgcgctgg     360

gcgcagccgc ttggccctca gccctctggc gcggcgccca cccgctgggt cccgccccgg     420

cagcgacgca gggataaccc gcggccgcgc ctgcccgctc gcacccctct cccgcgcccg     480

gttctccctc gcagcacctc gaagtgcgcc cctcgccctc ctgctcgcgc cccgccgcca     540

tggctgcctc ccccgcgcgg cctgctgtcc tggccctgac cgggctggcg ctgctcctgc     600

tcctgtgctg gggcccaggt gagcggggcg atgccaggct gattgatagc ggcaccaggg     660

gttggcccca tgtggcgctt ccatggtgcc cggggagagc gatcggtgat ggggtggcgg     720

cgtagggacc catccttagc ctaggcaggg ccaaggggtg gtagaggacc gggtctgggg     780

ttttgcactc gcacaagtgc gaagtggtgc ccttgtgagg gtggagagag taccggggtc     840

aaggggaggg tctcagatct cccgggcaac cttgggcaag gagggccagg cgtccgaagg     900

gtgggcaagt agtgtttcgt ggcttaacgt aaggggcaaa agggttgtgg gggctttacg     960

cagctggtaa cccagaggag gcaaatggca ccatcaccgc cattcccttc caggcagtcg    1020

cttagcaacc aagttgactt gcgcttccca gctgggtcaa gttgcaattt gcaaagctgg    1080

gcacaggcct cacaccccag agaggggaaa ggctcttaaa ggagtaatgc ttaatctttc    1140

ctgcttcgtg gagctcccag cgaatcggtt gaaaactctg aaattctctg ctcgctctct    1200

gtaaaac                                                              1207

<210>  2
<211>  1680
<212>  DNA
<213>  human

<400>  2
tggtccttaa agggtctgtc actcacctca cctatttaga tgaaattatc acagagacat      60

aaaaaacatc ctttgcaaca tgaaataaac aagtagcccc aactgcaatg tgtatcacca 120

gataaaaaag acgcactcag cctgtagaag gccaggtgtg gacggctggt gggtaagaat 180

tcttccaatg gcaggatgga agtatcacca atagtttgta actaagatac atactggcac 240

tcacttacat gattaataac cagaaactca gaaatactac acgctaccaa gatacttccc 300

tcgtctcctc tgtttcctta gaacataaag gctggcagcc ttgctagtcc ctcctgcgct 360

agactgcagg tagtatcctt tgtcctgcct attaagcgtc cctggccaat gcagagggga 420

cgatctgccg gcttcaggct ggcaggtgcg gggtgggctg tgtgctgagg accaggctgg 480

ccttctcgcg tcctctggcg acagaaatta agcaagtccg caaagaagca tattgcacct 540

tgagggagag gaagaaagag ttgcgggggc gcgtggggta gggcccggga ggcagtgggt 600

gttggcaaga aacggagtag gaggctctac agaggagact ggtccttggt taagtgtcac 660

agccgcagga tgggagggtg tgggaggggt ccgtggggcc aacacaggtg gcccctgctg 720

tgcgcggcga ggcaggcttg gaatgccttc tccccgaaca gagctagccc agcactgggg 780

gctggaggcg cggctctggt gggactgagg ccgggaagcg gcgccgggcg gggtggggac 840

tggggaggag gcggggctgg tgtgggggcc acagggaggc gggccgggtt taagggggtg 900

gctgtaggtg gggtctggga agttggcctc ggtggggaca gggacaggag aggtggggcc 960

gggactgggg aggtgggggg aaatgggggc gtggagaaac ggagcttggg gttggggacc 1020

tggggaagct gggtcctgac tggggacagg gaggcgaggc cgggcttggg gggtgtccag 1080

ggaggcaggg tgtgggaagg tctggggcgg ggaagtgggg tctgggctgg ggtgggagag 1140

ttggagtcca gaaggggacc gcggggtgga aggggtccgg gctgggaccg gggtaggcgg 1200

agtccaggag gggacgcagg gtgggcgggg tccgggctgg gtccgtgggg tgggcggggt 1260

ccgggctggg acgcggggtg ggcggggtcc gggctgggtc cgtggggtgg gcggggtccg 1320

ggctgggtcc gtggggtggg cggagtccag gaggggaccg cagggtgggc ggggtccggg 1380

ctgggtccgt ggggtgggcg gggtccgggc tgggtccgtg gggtgggcgg ggtccgggct 1440

gggaccgcgg ggtgggcggg gtccgggctg ggtccgtggg gtgggcgggg tccgggctgg 1500

gaccgcgggg tgggcggccc cgagagcgtc ccgcagtggc tggagccctg ggcgctgcaa 1560

agcgtgtccc gccgggtccc cgagcgtccc gcgccctcgc cccgccatgc tcctgctgct 1620

ggggctgtgc ctggggctgt ccctgtgtgt ggggtcgcag gaagaggcgc agagctgggg 1680

<210> 3
<211> 26
<212> DNA
<213> artificial

<220>
<223> primer

<400> 3
aggggtgggt tgtgtgttga ggatta 26

<210>  4
<211>  32
<212>  DNA
<213>  artificial

<220>
<223>  primer

<400>  4
cctactccct ttcttaccaa cacccactac ct                                    32


<210>  5
<211>  18
<212>  DNA
<213>  artificial

<220>
<223>  primer

<400>  5
ggttgtgtgt tgaggatt                                                    18


<210>  6
<211>  28
<212>  DNA
<213>  artificial

<220>
<223>  primer

<400>  6
ttagggaggt agtgggtgtt ggtaagaa                                         28


<210>  7
<211>  32
<212>  DNA
<213>  artificial

<220>
<223>  primer

<400>  7
ctctattcca aaaaaaaaca ttccaaacct ac                                    32


<210>  8
<211>  19
<212>  DNA
<213>  artificial

<220>
<223>  primer

<400>  8
aaaaaaaaac attccaaac                                                   19


<210>  9
<211>  19
<212>  DNA
<213>  artificial

<220>
<223>  primer

<400>  9
ggggagggag agaggattt                                                    19


<210>  10
<211>  26
<212>  DNA
<213>  artificial

<220>
<223>  primer

<400>  10
accccatcaa aaccaaaaca acaaac                                            26


<210>  11
<211>  18
<212>  DNA
<213>  artificial

<220>
<223>  human

<400>  11
gggagggaga gaggattt                                                     18


<210>  12
<211>  24
<212>  DNA
<213>  artificial

<220>
<223>  primer

<400>  12
ggtaagaaac ggagtaggag gttt                                              24


<210>  13
<211>  24
<212>  DNA
<213>  artificial

<220>
<223>  primer

<400>  13
aaccacctat attaacccca cgaa                                              24


<210>  14
<211>  30
<212>  DNA
<213>  artificial

<220>
<223>  primer

<400>  14
acaccctccc atcctacgac tataacactt                                        30


<210>  15
<211>  25
<212>  DNA
<213>  artificial

<220>
<223>  primer

<400>  15
caacacaaat aacccctact atacg                                          25


<210>  16
<211>  27
<212>  DNA
<213>  artificial

<220>
<223>  primer

<400>  16
ttttcggttt tagttttatt agagtcg                                        27


<210>  17
<211>  30
<212>  DNA
<213>  artificial

<220>
<223>  primer

<400>  17
acgaaacaaa cttaaaatac cttctccccg                                     30


<210>  18
<211>  24
<212>  DNA
<213>  artificial

<220>
<223>  primer

<400>  18
cgtggggtta atataggtgg tttt                                           24


<210>  19
<211>  26
<212>  DNA
<213>  artificial

<220>
<223>  primer

<400>  19
ccccaatact aaactaactc tattcg                                         26


<210>  20
<211>  25
<212>  DNA
<213>  artificial

<220>
<223>  primer

<400>  20
aaacattcca aacctacctc gccgc                                          25

<210>  21
<211>  24
<212>  DNA
<213>  artificial

<220>
<223>  primer

<400>  21
gagagaggat ttcggtggta ttcg                                              24


<210>  22
<211>  24
<212>  DNA
<213>  aartificial

<400>  22
gaattatccc tacgtcgcta ccga                                              24


<210>  23
<211>  26
<212>  DNA
<213>  artificial

<220>
<223>  primer

<400>  23
acgaaaccca acgaataaac gccgcg                                            26


<210>  24
<211>  24
<212>  DNA
<213>  artificial

<220>
<223>  primer

<400>  24
tcgtagtatt tcgaagtgcg tttt                                              24


<210>  25
<211>  24
<212>  DNA
<213>  artificial

<220>
<223>  primer

<400>  25
acataaaacc aacccctaat accg                                              24


<210>  26
<211>  24
<212>  DNA
<213>  artificial

<220>
<223>  primer

<400>  26
caacctaaca tcgccccgct cacc                                              24

<210>  27
<211>  24
<212>  DNA
<213>  artificial

<220>
<223>  primer

<400>  27
tttcgtagta tttcgaagtg cgtt                                              24


<210>  28
<211>  24
<212>  DNA
<213>  artificial

<220>
<223>  primer

<400>  28
gatcaaaacc aaaacaacaa accg                                              24


<210>  29
<211>  29
<212>  DNA
<213>  artificial

<220>
<223>  primer

<400>  29
aacaaccata acgacgaaac gcgaacaaa                                         29


<210>  30
<211>  24
<212>  DNA
<213>  artificial

<220>
<223>  primer

<400>  30
aaatctccga aacccaactt atcc                                              24


<210>  31
<211>  26
<212>  DNA
<213>  artificial

<220>
<223>  primer

<400>  31
tttaaggtta aggatgtttg gttacg                                            26


<210>  32
<211>  24
<212>  DNA
<213>  artificial

<220>
<223>  primer

<400>  32
cgctacgaac aacgctaacc acgc                                              24


<210>  33
<211>  28
<212>  DNA
<213>  artificial

<220>
<223>  primer

<400>  33
aacgatacca aactaattaa taacgaca                                          28


<210>  34
<211>  27
<212>  DNA
<213>  artificial

<220>
<223>  primer

<400>  34
gtattatttc gtatttgtgc gagtgta                                           27


<210>  35
<211>  30
<212>  DNA
<213>  artificial

<220>
<223>  primer

<400>  35
aaccccatat aacgcttcca taatacccga                                        30